(19) Europäisches Patentamt
European Patent Office
Office européen des brevets

(11) EP 4 545 064 A1

(12) EUROPEAN PATENT APPLICATION
published in accordance with Art. 153(4) EPC

(43) Date of publication:
**30.04.2025 Bulletin 2025/18**

(21) Application number: **23827012.8**

(22) Date of filing: **09.06.2023**

(51) International Patent Classification (IPC):
***A61K 8/49*** *(2006.01)* ***A61K 8/29*** *(2006.01)*
***A61K 8/35*** *(2006.01)* ***A61K 8/55*** *(2006.01)*
***A61K 8/92*** *(2006.01)* ***A61Q 1/04*** *(2006.01)*

(52) Cooperative Patent Classification (CPC):
**A61K 8/29; A61K 8/35; A61K 8/49; A61K 8/55; A61K 8/92; A61Q 1/04**

(86) International application number:
**PCT/JP2023/021469**

(87) International publication number:
**WO 2023/248825 (28.12.2023 Gazette 2023/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.06.2022 JP 2022102033**

(71) Applicant: **Shiseido Company, Ltd.**
**Chuo-ku**
**Tokyo 104-0061 (JP)**

(72) Inventors:
- **KANAZAWA, Marina**
  **Tokyo 104-0061 (JP)**
- **KOSAKA, Kyohei**
  **Tokyo 104-0061 (JP)**
- **CHIBA, Kiriko**
  **Tokyo 104-0061 (JP)**

(74) Representative: **Ter Meer Steinmeister & Partner Patentanwälte mbB**
**Nymphenburger Straße 4**
**80335 München (DE)**

# (54) COSMETIC PREPARATION

(57) Provided is a cosmetic, in which titanium dioxide particles and an organic pigment are used in combination, that suppresses fading of the organic pigment, i.e., has excellent fading resistance.

A cosmetic, comprising the following components: (a) an organic pigment, (b) titanium dioxide particles, (c) 4-tert-butyl-4'-methoxybenzoylmethane, (d) a phospholipid and/or hydrogenated product thereof, and (e) an oil, wherein a ratio of a content of the component (d) relative to a content of the component (c) is less than 1.0.

EP 4 545 064 A1

## Description

FIELD

The present invention relates to a cosmetic.

BACKGROUND

Cosmetics (cosmetic preparations) such as makeup cosmetics, for the purpose of fulfilling the aesthetic functions thereof, are commonly blended therein various pigments.

These pigments, however, are very unstable against ultraviolet rays and oxidation, and there is a problem that the color tone of a cosmetic is changed or damaged, lowering the quality of the cosmetic. Particularly, red organic pigments often used in cosmetics such as lipstick and eye shadow decolor significantly, and such decoloration has a large effect on the color tones of the cosmetics.

In cosmetics comprising organic pigments, improving light fastness, i.e., resistance to decoloration, of organic pigments, particularly red organic pigments, is strongly desired.

In response to such a problem, for example, PTL 1 discloses a makeup cosmetic comprising an organic pigment in which 4-tert-butyl-4'-methoxybenzoylmethane, calcium stearate, and a phospholipid and/or hydrogenated product thereof are blended.

[CITATION LIST]

[PATENT LITERATURE]

[PTL 1] Japanese Unexamined Patent Publication (Kokai) No.2005-200378

SUMMARY

[TECHNICAL PROBLEM]

Titanium dioxide particles as a white pigment or an ultraviolet scattering agent are often blended in cosmetics such as lipstick and eye shadow.

The present inventors have discovered that in cosmetics in which an organic pigment, particularly Red No. 104 (1), is blended with titanium dioxide particles, fading of the Red No. 104 (1) is significant.

The present invention aims to improve the above circumstances, and an object thereof is to provide a cosmetic, in which titanium dioxide particles and an organic pigment are used in combination, that suppresses fading of the organic pigment, i.e., has excellent fading resistance.

[SOLUTION TO PROBLEM]

The present invention for achieving the above object is as follows.

<Aspect 1>

A cosmetic, comprising components below:

(a) an organic pigment,
(b) titanium dioxide particles,
(c) 4-tert-butyl-4'-methoxybenzoylmethane,
(d) a phospholipid and/or hydrogenated product thereof, and
(e) an oil,

wherein a ratio of a content of the component (d) relative to a content of the component (c) is less than 1.0.

<Aspect 2>

The cosmetic according to Aspect 1, wherein the organic pigment comprises Red No. 104 (1).

<Aspect 3>

The cosmetic according to Aspect 1 or 2, wherein the ratio is 0.5 or less.

<Aspect 4>

The cosmetic according to any one of Aspects 1 to 3, wherein a content of the 4-tert-butyl-4'-methoxybenzoylmethane is 0.1 to 10% by mass.

<Aspect 5>

The cosmetic according to any one of Aspects 1 to 4, wherein a content of the phospholipid and/or hydrogenated product thereof is 0.01 to 5.0% by mass.

<Aspect 6>

The cosmetic according to any one of Aspects 1 to 5, wherein a content of the titanium dioxide particles is 0.1 to 20% by mass.

<Aspect 7>

The cosmetic according to any one of Aspects 1 to 6, wherein a content of the organic pigment is 0.001 to 20% by mass.

<Aspect 8>

The cosmetic according to any one of Aspects 1 to 7, wherein an apparent iodine value of the oil is 6.0 to 30.0.

<Aspect 9>

The cosmetic according to any one of Aspects 1 to 8, which is an oily cosmetic.

<Aspect 10>

The cosmetic according to any one of Aspects 1 to 9, which is a lip cosmetic.

[ADVANTAGEOUS EFFECTS OF INVENTION]

According to the present invention, fading of an organic pigment in a cosmetic, in which titanium dioxide particles and the organic pigment are used in combination, can be suppressed.

DESCRIPTION OF EMBODIMENTS

Hereinafter, embodiments of the present invention will be described in detail. The present invention is not limited to the following embodiments, and various modifications can be made within the scope of the invention.

<<Cosmetic>>

The cosmetic of the pr esent invention is

a cosmetic, comprising the following components:

(a) an organic pigment,
(b) titanium dioxide particles,
(c) 4-tert-butyl-4'-methoxybenzoylmethane,
(d) a phospholipid and/or hydrogenated product thereof, and
(e) an oil,

wherein a ratio of a content of the component (d) relative to a content of the component (c) is less than 1.0.

Without being bound by theory, the fading of the organic pigment when titanium dioxide particles and the organic pigment are used in combination is presumably due to the following mechanism. Specifically, it is considered that titanium dioxide particles in the cosmetic generate radicals when irradiated with light, and these radicals attack organic pigments such as Red No. 104 (1), resulting in fading.

In response to such a fading problem, the present inventors have discovered that by containing specific components, 4-tert-butyl-4'-methoxybenzoylmethane and a phospholipid and/or hydrogenated product thereof, in a specific mass content ratio in the cosmetic of the present invention, fading of organic pigments such as Red No. 104 (1) can be suppressed.

Without being bound by theory, it is considered that the radicals from the titanium dioxide particles are transmitted by 4-tert-butyl-4'-methoxybenzoylmethane, the phospholipid and/or hydrogenated product thereof deactivates the transmitted radicals, and thereby fading of organic pigments such as Red No. 104 (1) can be suppressed.

Further intensive studies by the present inventors have revealed that when the ratio of the content of component (d) (phospholipid and/or hydrogenated product thereof) relative to the content of component (c) (4-tert-butyl-4'-methoxybenzoylmethane) is less than 1.0, fading of organic pigments such as Red No. 104 (1) can particularly be suppressed. When the ratio is 1.0 or greater, specifically when the content of the phospholipid and/or hydrogenated product thereof is the same or higher than the content of 4-tert-butyl-4'-methoxybenzoylmethane, it is presumed that the content of 4-tert-butyl-4'-methoxybenzoylmethane is relatively too low, whereby the transmission of radicals from the titanium dioxide particles is not sufficiently carried out, and/or the content of the phospholipid and/or hydrogenated product thereof is relatively too high, which in turn imparts a certain effect on nearby organic pigments such as Red No. 104 (1) when deactivating the radicals to accelerate fading.

The "ratio of a content of a phospholipid and/or hydrogenated product thereof relative to a content of 4-tert-butyl-4'-methoxybenzoylmethane" in the present invention can be calculated by the formula "mass content of phospholipid and/or hydrogenated product thereof / mass content of 4-tert-butyl-4'-methoxybenzoylmethane". When the component (d) comprises two or more phospholipids and/or hydrogenated products thereof, the total mass content thereof is used for the calculation.

In the cosmetic of the present invention, the ratio of the content of the phospholipid and/or hydrogenated product thereof relative to the content of 4-tert-butyl-4'-methoxybenzoylmethane is less than 1.0, and more specifically, may be 0.95 or less, 0.90 or less, 0.85 or less, 0.80 or less, 0.75 or less, 0.70 or less, 0.65 or less, 0.60 or less, 0.55 or less, 0.50 or less, 0.45 or less, 0.40 or less, 0.35 or less, 0.30 or less, 0.25 or less, 0.20 or less, 0.15 or less, 0.10 or less, 0.09 or less, 0.08 or less, 0.07 or less, 0.06 or less, 0.05 or less, or 0.04 or less. The lower limit of the ratio needs only to be greater than 0 and is not particularly limited, and for example, may be 0.01 or greater, 0.02 or greater, 0.03 or greater, or 0.04 or greater.

Hereinafter, each component that can be contained in the cosmetic of the present invention will be described in detail.

<Component (a) Organic pigment>

The organic pigment that can be contained in the cosmetic of the present invention is not particularly limited, and may be any organic pigment that can generally be used in cosmetics. More specifically, examples include Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, Blue No. 404, Red No. 3, Red No. 104 (1), Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1. One or more of these organic pigments can be contained in the cosmetic of the present invention. Particularly, when a red organic pigment, especially Red No. 104 (1), is contained, the effect of the present invention is more remarkable. The Red No. 104 (1) (or written as "Red No. 104") herein is a colorant that impart a pink color, and is also called "Phloxine B".

In the cosmetic of the present invention, the content of the organic pigment is not particularly limited, and for example, may be 0.001% by mass or greater, 0.005% by mass or greater, 0.01% by mass or greater, 0.05% by mass or greater, 0.10% by mass or greater, 0.50% by mass or greater, 1.0% by mass or greater, 1.1% by mass or greater, 1.2% by mass or greater, 1.3% by mass or greater, 1.4% by mass or greater, 1.5% by mass or greater, 1.6% by mass or greater, 1.7% by mass or greater, 1.8% by mass or greater, 1.9% by mass or greater, 2.0% by mass or greater, 2.1% by mass or greater, 2.2% by mass or greater, 2.3% by mass or greater, 2.4% by mass or greater, or 2.5% by mass or greater relative to the entire cosmetic, and may be 20% by mass or less, 15% by mass or less, 10% by mass or less, or 5.0% by mass or less.

<Component (b) Titanium dioxide particles>

The titanium dioxide particles that can be contained in the cosmetic of the present invention are not particularly

limited, and may be any titanium dioxide particles that can generally be used in cosmetics. The titanium dioxide particles, for example, may be ones used as an inorganic pigment (for example, white pigment) or an ultraviolet scattering agent.

In the present invention, the titanium dioxide particles may be used as they are, or hydrophobically treated ones may be used. Therefore, at least a portion of the surfaces of the titanium dioxide particles may be coated with another material, for example, silicone.

In the cosmetic of the present invention, the content of the titanium dioxide particles (if the titanium dioxide particles are coated, the content of the titanium dioxide particles includes the weight of the coating treatment material) is not particularly limited, and for example, may be 0.01% by mass or greater, 0.05% by mass or greater, 0.10% by mass or greater, 0.50% by mass or greater, 1.0% by mass or greater, 1.1% by mass or greater, 1.2% by mass or greater, 1.3% by mass or greater, 1.4% by mass or greater, 1.5% by mass or greater, 1.6% by mass or greater, 1.7% by mass or greater, 1.8% by mass or greater, 1.9% by mass or greater, 2.0% by mass or greater, 2.1% by mass or greater, 2.2% by mass or greater, 2.3% by mass or greater, 2.4% by mass or greater, or 2.5% by mass or greater relative to the entire cosmetic, and may be 20% by mass or less, 15% by mass or less, 10% by mass or less, or 5.0% by mass or less.

When the titanium dioxide particles are coated with a coating treatment material that is an organic material and/or an inorganic material, generally, the coating amount of the coating treatment material is often within 20% by mass of the entirety of the titanium dioxide particles.

<Component (c) 4-Tert-butyl-4'-methoxybenzoylmethane>

4-Tert-butyl-4'-methoxybenzoylmethane is a dibenzoylmethane derivative represented by the following structural formula (I), and is generally used in foundation cosmetics and makeup cosmetics as an oil-soluble ultraviolet absorber:

[Chem. 1]

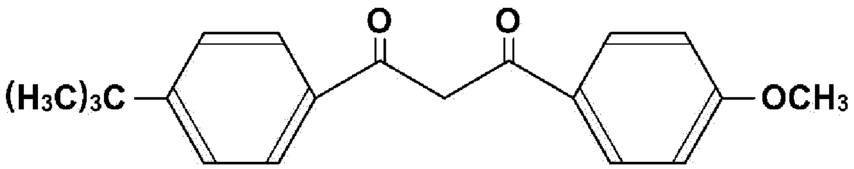

(I)

The 4-tert-butyl-4'-methoxybenzoylmethane is commercially available, for example, a commercially available product such as Parsol 1789 (manufactured by Givaudan) can be used in the present invention.

In the cosmetic of the present invention, the content of 4-tert-butyl-4'-methoxybenzoylmethane is not particularly limited. For example, from the viewpoint of better demonstrating the effect of the present invention, the content of the 4-tert-butyl-4'-methoxybenzoylmethane may be 0.1% by mass or greater, 0.2% by mass or greater, 0.3% by mass or greater, 0.4% by mass or greater, 0.5% by mass or greater, 0.6% by mass or greater, 0.7% by mass or greater, 0.8% by mass or greater, 0.9% by mass or greater, 1.0% by mass or greater, 1.1% by mass or greater, 1.2% by mass or greater, 1.3% by mass or greater, 1.4% by mass or greater, 1.5% by mass or greater, 1.6% by mass or greater, 1.7% by mass or greater, 1.8% by mass or greater, 1.9% by mass or greater, 2.0% by mass or greater, 2.1% by mass or greater, 2.2% by mass or greater, 2.3% by mass or greater, 2.4% by mass or greater, or 2.5% by mass or greater relative to the entire cosmetic. From the viewpoint of safety and usability, the content of 4-tert-butyl-4'-methoxybenzoylmethane may be 10% by mass or less, 9.5% by mass or less, 9.0% by mass or less, 8.5% by mass or less, 8.0% by mass or less, 7.5% by mass or less, 7.0% by mass or less, 6.5% by mass or less, 6.0% by mass or less, 5.5% by mass or less, 5.0% by mass or less, 4.5% by mass or less, 4.0% by mass or less, 3.5% by mass or less, 3.0% by mass or less, or 2.5% by mass or less relative to the entire cosmetic.

<Component (d) Phospholipid and/or hydrogenated product thereof>

In the present invention, the phospholipid and/or hydrogenated product thereof may be a natural or synthetic phospholipid, or a hydrogenated product thereof. The phospholipid may be a substance used as a raw material of the cosmetic, and examples include phosphatidylcholine, phosphatidylethanolamine, phosphatidylserine, phosphatidylinositol, phosphatidic acid, and phosphatidylglycerol; and lyso compounds thereof.

The phospholipid and/or hydrogenated product thereof that can be contained in the cosmetic of the present invention is not particularly limited, and examples include natural phospholipid-containing substances extracted from plants such as soybean, corn, and rapeseed and from microbes, and hydrogenated products thereof; and mixtures of one

or more phospholipids, such as phosphatidylcholine, phosphatidylethanolamine, phosphatidylserine, phosphatidylinositol, phosphatidic acid, and phosphatidylglycerol, obtained by synthesis or isolated or fractionated from nature.

The phospholipid and/or hydrogenated product thereof used in the present invention may comprise preferably 10% by mass or greater, more preferably 15% by mass or greater, and even more preferably 18% by mass or greater of phosphatidylethanolamine. For example, soybean lecithin, particularly Basis LP-20H (18 to 25% by mass in phosphatidylethanolamine content), which is a hydrogenated soybean phospholipid commercially available from Nisshin OilliO Group, Ltd., can be suitably used in the present invention.

In the cosmetic of the present invention, the content of the phospholipid and/or hydrogenated product thereof is not particularly limited, but from the viewpoint of better demonstrating the effect of the present invention, may be 0.01% by mass or greater, 0.02% by mass or greater, 0.03% by mass or greater, 0.04% by mass or greater, 0.05% by mass or greater, 0.06% by mass or greater, 0.07% by mass or greater, 0.08% by mass or greater, 0.09% by mass or greater, 0.10% by mass or greater, 0.11% by mass or greater, 0.12% by mass or greater, 0.13% by mass or greater, 0.14% by mass or greater, 0.15% by mass or greater, 0.16% by mass or greater, 0.17% by mass or greater, 0.18% by mass or greater, 0.19% by mass or greater, or 0.20% by mass or greater relative to the entire cosmetic. From the viewpoint of usability, the content of the phospholipid and/or hydrogenated product thereof may be 5.0% by mass or less, 4.0% by mass or less, 3.0% by mass or less, 2.0% by mass or less, 1.0% by mass or less, 0.9% by mass or less, 0.8% by mass or less, 0.7% by mass or less, 0.6% by mass or less, or 0.5% by mass or less relative to the entire cosmetic. The "content of phospholipid and/or hydrogenated product thereof" in the present invention, when the cosmetic of the present invention comprises both a phospholipid and a hydrogenated product thereof, refers to the total content thereof.

<Component (e) Oil>

The oil that can be contained in the cosmetic of the present invention is not particularly limited, and may be any oil that can generally be used in cosmetics. The oil, for example, may be a vegetable oil or a synthetic oil, and the state thereof, for example, may be solid, semi-solid, liquid, or volatile. More specifically, examples of the oil include, but are not limited to, hydrocarbon oils, oils and fats, waxes, hardened oils, fatty acids, higher alcohols, silicone oils, fluorine-based oils, oily gelling agents, and polar oils. The oils can be used independently or in combinations of two or more.

Examples of hydrocarbon oils can include liquid paraffin, heavy liquid isoparaffin, $\alpha$-olefin oligomers, squalane, petrolatum, polyisobutene, hydrogenated polyisobutene, polybutene, hydrogenated polybutene, polydecene, and hydrogenated polydecene.

Examples of oils and fats can include olive oil, castor oil, jojoba oil, and macadamia nut oil.

Examples of waxes can include paraffin wax, ceresin wax, microcrystalline wax, polyethylene wax, montan wax, Fischer-Tropsch wax, carnauba wax, candelilla wax, Japan wax, and beeswax.

Examples of fatty acids can include stearic acid, lauric acid, myristic acid, behenic acid, isostearic acid, and oleic acid.

Examples of higher alcohols can include stearyl alcohol, cetyl alcohol, lauryl alcohol, oleyl alcohol, isostearyl alcohol, behenyl alcohol, and octyldodecanol.

Examples of silicones can include low-polymerization dimethylpolysiloxane, high-polymerization dimethylpolysiloxane, methylphenyl polysiloxane, decamethylcyclopentasiloxane, octamethylcyclotetrasiloxane, polyether-modified polysiloxane, polyoxyalkylene-alkylmethylpolysiloxane-methylpolysiloxane copolymer, alkoxy-modified polysiloxane, crosslinked organopolysiloxane, and fluorine-modified polysiloxane.

Examples of fluorine-based oils can include perfluorodecane, perfluorooctane, and perfluoropolyether.

Examples of oily gelling agents can include sucrose fatty acid ester, starch fatty acid ester, aluminum 12-hydroxystearate, and calcium stearate.

Examples of polar oils can include polar oils in which the IOB is 0.10 or greater, 0.15 or greater, 0.20 or greater, 0.22 or greater, or 0.24 or greater. The upper limit value of IOB is not particularly limited, and for example, can be 0.50 or less, 0.45 or less, or 0.40 or less. The IOB value herein is an abbreviation for Inorganic/Organic Balance, wherein the value represents a ratio of an inorganic value relative to an organic value, and is an index indicating the degree of polarity of an organic compound. The IOB value is specifically represented as IOB value = inorganic value / organic value. Each of the "inorganic value" and the "organic value" are set according to various atoms or functional groups, for example, an "organic value" of 20 for one carbon atom and an "inorganic value" of 100 for one hydroxyl group in a molecule. The IOB value of an organic compound can be calculated by integrating the "inorganic values" and "organic values" of all atoms and functional groups in the organic compound (refer to, for example, Koda, Yoshio et al., "Organic Conceptual Diagrams -Fundamentals and Applications-", pp. 11 to 17, Sankyo Publishing Co., Ltd., 1984).

Examples of such polar oils can include neopentyl glycol dicaprate, glyceryl tri-2-ethylhexanoate, pentaerythrityl tetra-2-ethylhexanoate, glyceryl tri(caprylate/caprate), diethylhexyl sebacate, octyldodecanol, glyceryl diisostearate, diglyceryl triisostearate, diisostearyl malate, trimethylolpropane tri-2-ethylhexanoate, oxystearyl oxystearate, pentaerythrityl tetra(ethylhexanoate/benzoate), trioctanoin, pentaerthyrityl tetraoctanoate, dipentaerythrityl hexahydrostearate,

castor oil, diisopropyl sebacate, pentaerythrityl tetraoctanoate, and trimethylolpropane triisostearate.

Among the oils mentioned above, from the viewpoint of better transmitting radicals from the titanium dioxide particles, it is particularly preferable to include oils capable of dissolving 4-tert-butyl-4'-methoxybenzoylmethane. For example, the oil preferably comprises at least one selected from hydrogenated polyisobutene, microcrystalline wax, polyethylene wax, octyldodecanol, diisostearyl malate, and neopentyl glycol dicaprate.

In the present invention, the apparent iodine value of the oil may be 6.0 to 30.0. The "apparent iodine value of oil" is used as a measure of the unsaturated portion contained in an oil. A higher apparent iodine value of the oil indicates a higher ratio of the unsaturated portion relative to the entire cosmetic.

When the cosmetic comprises only one oil, the "apparent iodine value of oil" is the product of the iodine value of the oil and the weight ratio of the oil relative to the entire cosmetic. Specifically, for example, when only an oil A is contained as the oil in the cosmetic, if the iodine value of the oil A is "a" and the content ratio of the oil A relative to the entire cosmetic is "x% by mass", the "apparent iodine value of oil" of the cosmetic can be determined from the following formula (1):

$$\text{Apparent iodine value of oil} = a \times x\% \quad \cdots (1)$$

When the cosmetic comprises two or more oils, the "apparent iodine value of oil" is a value obtained by totaling the product of the iodine value of each oil and the weight ratio of the oil relative to the entire cosmetic for all oils. Specifically, for example, when two different oils A and B are contained in the cosmetic, if the iodine value of the oil A is "a"; the content ratio of the oil A relative to the entire cosmetic is "x% by mass"; the iodine value of the oil B is "b"; and the content ratio of the oil B relative to the entire cosmetic is "y% by mass", the "apparent iodine value of oil" of the cosmetic can be determined from the following formula (2):

$$\text{Apparent iodine value of oil} = a \times x\% + b \times x\% \quad \cdots (2)$$

The iodine value of each oil is the number of grams of iodine ($I_2$) that can be added to a 100 g oil sample, and can be measured by a test in accordance with Japanese Industrial Standards (JIS) K0070:1992.

In the present invention, a "saturated oil" can be defined as an oil having an iodine value of 5.0 or less, and an "unsaturated oil" can be defined as an oil having an iodine value of greater than 5.0.

In the cosmetic of the present invention, the content of the oil is not particularly limited, and can be appropriately set according to the formulation type of the intended cosmetic. For example the content of the oil may be 1.0% by mass or greater, 5.0% by mass or greater, 10% by mass or greater, 20% by mass or greater, 30% by mass or greater, 40% by mass or greater, 50% by mass or greater, 60% by mass or greater, 70% by mass or greater, 80% by mass or greater, or 90% by mass or greater relative to the entire cosmetic, and may be 99% by mass or less, 95% by mass or less, or 90% by mass or less.

<Additional optional component>

The cosmetic of the present invention, in addition to the components mentioned above, may further comprise one or more additional optional components, as long as the effect of the present invention is not impaired.

In the present invention, examples of additional optional components include, but are not limited to, surfactants, water, humectants, polymers, thickeners, coating agents, ultraviolet absorbers, metal-ion sequestrants, lower alcohols, polyhydric alcohols, saccharides, amino acids, organic amines, polymer emulsions, pH modifiers, skin nutrients, vitamins, antioxidants, antioxidant aids, and perfumes. The additional optional component can be appropriately blended as long as the effect of the present invention is not impaired, and manufacturing can be carried out by a conventional method according to the type of the intended cosmetic. In addition to the organic pigment mentioned above, additional optional coloring materials that are generally used in cosmetics may be contained as long as the effect of the present invention is not impaired.

<Dosage form and application of cosmetic>

The cosmetic of the present invention is not particularly limited in the dosage form thereof, and examples include any dosage form such as oily-type, emulsion-type, and powder-type. Since fading of organic pigments, in particular, is likely a problem in oily-type cosmetics among these dosage forms, it is preferable that the cosmetic of the present invention be an oily cosmetic. An oily cosmetic refers to a cosmetic in which a liquid, semi-solid, or solid oil agent or oil-soluble compound forms a continuous phase.

The cosmetic of the present invention is not particularly limited in the application thereof, and for example, is used as a lip cosmetic.

Examples of lip cosmetics include lipstick, gloss, lip treatment, lip cream, and lip primer. Among these, lipstick and lip cream are particularly preferable.

In addition thereto, the cosmetic of the present invention can be used as, for example, foundation, blush, eye shadow, eyeliner, mascara, eyebrow, nail enamel, and nail treatment.

EXAMPLES

The present invention will be further described in detail below with reference to the Examples. However, the present invention is not limited thereto. Hereinafter, blending amounts are shown in % by mass unless specified otherwise.

<<Examples 1 to 6 and Comparative Examples 1 to 11>>

Lipstick samples of Examples 1 to 6 and Comparative Examples 1 to 11 were produced based on the components and the respective blending amounts shown in Table 1 below.

(Method of evaluating fading)

Each produced sample was irradiated using a xenon fade meter (manufactured by Suga Test Instruments Co., Ltd.) at 120 MJ/$m^2$ at 25°C for 30 h, and the color difference (ΔE) before and after irradiation in each sample was measured. The fading of organic pigment of each sample was evaluated, and the evaluation results are shown in Table 1.

More specifically, based on the color difference ($\Delta E_1$) in Comparative Example 1, when the color difference in other samples (Examples 1 to 6 and Comparative Example 2 to 11) is "$\Delta E_x$", the "fading suppression rate" in each of the other samples was determined by the following formula. Note that a higher value of "fading suppression rate" means less fading of a sample, i.e., having excellent fading resistance.

$$\text{Fading suppression rate of sample} = [(\Delta E_1 - \Delta E_x)/\Delta E_1] \times 100\%$$

[Table 1]

(Table 1)

| Component | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Example 1 | Example 2 | Example 3 | Example 4 | Example 5 | Example 6 | Comparative Example 10 | Comparative Example 11 |
|---|---|---|---|---|---|---|---|---|---|---|---|---|---|---|---|---|---|
| Mixture of polyethylene wax and microcrystalline wax | 10.0 | 10.0 | 10.0 | 10.0 | 10.0 | 10.0 | 10.0 | 10.0 | 10.0 | 10.0 | 10.0 | 10.0 | 10.0 | 10.0 | 10.0 | 10.0 | 10.0 |
| Red No. 104 (1) ··· *1 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 |
| Titanium dioxide particles ··· *2 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 |
| Diisostearyl malate | 85.0 | 84.95 | 84.8 | 84.5 | 84.0 | 84.5 | 84.0 | 83.5 | 84.8 | 84.4 | 83.9 | 82.4 | 83.8 | 83.95 | 83.5 | 83.0 | 84.75 |
| Calcium stearate | - | - | - | - | - | - | - | - | - | - | - | - | - | - | - | - | 0.05 |
| 4-Tert-butyl-4'-methoxybenzoylmethane | - | - | - | - | - | 0.5% | 1.0% | 1.5 | 0.1 | 0.5 | 1.0 | 2.5 | 1.0 | 1.0 | 1.0 | 1.0 | 0.1 |
| Phospholipid and/or hydrogenated product thereof ··· *3 | - | 0.05 | 0.2 | 0.5 | 1.0 | - | - | - | 0.1 | 0.1 | 0.1 | 0.1 | 0.2 | 0.05 | 0.5 | 1.0 | 0.1 |
| Total | 100 | 100 | 100 | 100 | 100 | 100 | 100 | 100 | 100 | 100 | 100 | 100 | 100 | 100 | 100 | 100 | 100 |
| Ratio of content of phospholipid and/or hydrogenated product thereof relative to content of 4-tert-butyl-4'-methoxybenzoylmethane | - | - | - | - | - | 0 | 0 | 0 | 1.0 | 0.2 | 0.1 | 0.04 | 0.2 | 0.05 | 0.5 | 1.0 | 1.0 |
| After Xe irradiation (25°C, 30 h) ΔE | 39.7 | 38.6 | 30.1 | 30.1 | 35.9 | 34.9 | 30.9 | 31.1 | 33.4 | 25.7 | 12.8 | 23.4 | 15.2 | 17.5 | 28.8 | 33.1 | 31.4 |
| Fading suppression rate | reference (0%) | 2.7% | 24.2% | 24.2% | 9.6% | 12.0% | 22.3% | 21.8% | 15.9% | 35.3% | 67.8% | 41.1% | 61.7% | 55.9% | 27.4% | 16.6% | 21.0% |

*1: Red No. 104 (1) is a 50% by mass oil dispersion. Note that blending amount is calculated based on solid portion of Red No. 104 (1).
*2: Silicone-treated titanium oxide is a 65% oil dispersion. Note that blending amount is calculated based on solid portion of silicone-treated titanium dioxide.
*3: Basis LP-20H (manufactured by Nisshin OilliO Group, Ltd.)

As is clear from Table 1, the fading suppression rates in the samples of Examples 1 to 6, wherein each of the ratios of the content of the phospholipid and/or hydrogenated product thereof relative to the content of 4-tert-butyl-4'-methoxybenzoylmethane was less than 1.0, were higher than the fading suppression rates in the Comparative Examples. This means that the samples of Examples 1 to 6 had excellent fading resistance.

Comparative Example 11 was a sample in which "calcium stearate" described in PTL 1 was blended. However, since the ratio of the content of the phospholipid and/or hydrogenated product thereof relative to the content of 4-tert-butyl-4'-methoxybenzoylmethane was 1.0, fading resistance was found to be inferior.

<<Reference Examples 1 and 2>>

To verify fading of organic pigment due to titanium dioxide particles, lipstick samples of Reference Examples 1 and 2 were produced based on the components and blending amounts shown in Table 2 below. Fading in the samples of Reference Examples 1 and 2 was evaluated based on Comparative Example 1, in the same manner as the above evaluation method, and the results are shown in Table 2. For comparison, the results of Comparative Example 9 are also shown in Table 2.

[Table 2]

(Table 2)

| Component | Comparative Example 1 | Reference Example 1 | Comparative Example 9 | Reference Example 2 |
|---|---|---|---|---|
| Mixture of polyethylene wax and microcrystalline wax | 10.0 | 10.0 | 10.0 | 10.0 |
| Red No. 104 (1) ··· *1 | 2.5 | 5.0 | 2.5 | 2.5 |
| Titanium dioxide particles ··· *2 | 2.5 | - | 2.5 | - |
| Diisostearyl malate | 85.0 | 85.0 | 84.8 | 87.3 |
| 4-Tert-butyl-4'-methoxybenzoylmethane | - | - | 0.1 | 0.1 |
| Phospholipid and/or hydrogenated product thereof ··· *3 | - | - | 0.1 | 0.1 |
| Total | 100 | 100 | 100 | 100 |
| After Xe irradiation (25°C, 30 h) ΔE | 39.7 | 1.6 | 33.4 | 3.0 |
| Fading suppression rate | reference (0%) | 96% | 15.9% | 92.4% |

*1: Red No. 104 (1) is a 50% by mass oil dispersion. Note that blending amount is calculated based on solid portion of Red No. 104 (1).
*2: Silicone-treated titanium oxide is a 65% oil dispersion. Note that blending amount is calculated based on solid portion of silicone-treated titanium dioxide.
*3: Basis LP-20H (manufactured by Nisshin OilliO Group, Ltd.)

From Table 2, it is understood that Reference Examples 1 and 2, which comprise no titanium dioxide particles, had a higher fading suppression rate, i.e., less fading of the organic pigment, compared to the similar Comparative Examples 1 and 9 except for comprising titanium dioxide particles.

Therefore, it was suggested that in cosmetics wherein titanium dioxide particles and an organic pigment are used in combination, fading of the organic pigment is due to the presence of titanium dioxide particles.

<<Reference Examples 3 to 12>>

It has been known that 4-tert-butyl-4'-methoxybenzoylmethane can be used as an ultraviolet absorber. Therefore, the fading suppression effect of 4-tert-butyl-4'-methoxybenzoylmethane only and the fading suppression effect when other ultraviolet absorbers were used in place of 4-tert-butyl-4'-methoxybenzoylmethane were verified.

More specifically, lipstick samples of Reference Examples 3 to 12 were produced based on the components and

blending amounts shown in Table 3 below. Fading in the samples of Reference Examples 3 to 12 was evaluated based on Comparative Example 1, in the same manner as the above evaluation method. Results are shown in Table 3.

[Table 3]

(Table 3)

| Component | Comparative Example 1 | Reference Example 3 | Reference Example 4 | Reference Example 5 | Reference Example 6 | Reference Example 7 | Reference Example 8 | Reference Example 9 | Reference Example 10 | Reference Example 11 | Reference Example 12 |
|---|---|---|---|---|---|---|---|---|---|---|---|
| Mixture of polyethylene wax and microcrystalline wax | 10 | 10 | 10 | 10 | 10 | 10 | 10 | 10 | 10 | 10 | 10 |
| Red No. 104 (1) … *1 | 2.5 | 2.5 | 2.5 | 2.5 | 2.5 | 2.5 | 2.5 | 2.5 | 2.5 | 2.5 | 2.5 |
| Titanium dioxide particles … *2 | 2.5 | 2.5 | 2.5 | 2.5 | 2.5 | 2.5 | 2.5 | 2.5 | 2.5 | 2.5 | 2.5 |
| Diisostearyl malate | 85.0 | 84.0 | 84.0 | 84.0 | 84.0 | 84.0 | 84.0 | 84.0 | 84.0 | 84.0 | 84.0 |
| 4-Tert-butyl-4'-methoxybenzoylmethane | - | 1.0 | - | - | - | - | - | - | - | - | - |
| Ethylhexyl methoxycinnamate | - | - | 1.0 | - | - | - | - | - | - | - | - |
| Homosalate | - | - | - | 1.0 | - | - | - | - | - | - | - |
| Ethylhexyl salicylate | - | - | - | - | 1.0 | - | - | - | - | - | - |
| Diethylamino hydroxybenzoyl hexyl benzoate | - | - | - | - | - | 1.0 | - | - | - | - | - |
| Bis-ethylhexyloxyphenol methoxyphenyl triazine | - | - | - | - | - | - | 1.0 | - | - | - | - |
| Oxybenzone-3 | - | - | - | - | - | - | - | 1.0 | - | - | - |
| Polysilicone-15 | - | - | - | - | - | - | - | - | 1.0 | - | - |
| Ethylhexyl triazone | - | - | - | - | - | - | - | - | - | 1.0 | - |
| Octocrylene | - | - | - | - | - | - | - | - | - | - | 1.0 |
| Total | 100 | 100 | 100 | 100 | 100 | 100 | 100 | 100 | 100 | 100 | 100 |
| After Xe irradiation (25°C, 30 h) ΔE | 39.7 | 30.9 | 39.6 | 40.1 | 43.6 | 44.0 | 47.3 | 47.4 | 47.6 | 48.1 | 48.4 |

(continued)

| Component | Comparative Example 1 | Reference Example 3 | Reference Example 4 | Reference Example 5 | Reference Example 6 | Reference Example 7 | Reference Example 8 | Reference Example 9 | Reference Example 10 | Reference Example 11 | Reference Example 12 |
|---|---|---|---|---|---|---|---|---|---|---|---|
| Fading suppression rate | reference (0%) | 22.2% | 0.2% | -1.1% | -9.7% | -10.7% | -19.1% | -19.3% | -19.8% | -21.1% | -21.8% |

*1: Red No. 104 (1) is a 50% by mass oil dispersion. Note that blending amount is calculated based on solid portion of Red No. 104 (1).
*2: Silicone-treated titanium oxide is a 65% oil dispersion. Note that blending amount is calculated based on solid portion of silicone-treated titanium dioxide.

In Table 3, the fading suppression rate having a negative value means that the fading suppression effect was inferior to that of Comparative Example 1, i.e., fading had progressed. As is clear from Table 3, Reference Example 3 containing 4-tert-butyl-4'-methoxybenzoylmethane had a remarkably excellent fading suppression effect, compared to Reference Examples 4 to 12 containing other ultraviolet absorbers, therefore suggesting that the fading suppression effect of 4-tert-butyl-4'-methoxybenzoylmethane was not merely an effect of any ultraviolet absorber.
Although Reference Example 3 contained 4-tert-butyl-4'-methoxybenzoylmethane, the fading suppression effect had room for further improvement. In contrast, as indicated in Table 1, Examples 1 to 6 had an excellent fading suppression effect compared to Reference Example 3.

<<Formulation Example>>

A Formulation Example of the cosmetic of the present invention is exemplified below. However, the present invention is not limited thereto.

[Table 4]

| (Table 4 Formulation Example) | |
|---|---|
| Component | Blending amount |
| Mixture of polyethylene wax and microcrystalline wax (iodine value of oil: about 0) | 10.0 |
| Red No. 104 (1) ··· *1 | 2.5 |
| Titanium dioxide particles ··· *2 | 2.5 |
| Diisostearyl malate (iodine value: 2.0) | 63.9 |
| Olive fruit oil (iodine value: 83.5) | 20.0 |
| 4-Tert-butyl-4'-methoxybenzoylmethane | 1.0 |
| Phospholipid and/or hydrogenated product thereof ··· *3 | 0.1 |
| Total | 100 |
| *1: Red No. 104 (1) is a 50% by mass oil dispersion. Note that blending amount is calculated based on solid portion of Red No. 104 (1). Iodine value of the oil is about 0.<br>*2: Silicone-treated titanium oxide is a 65% oil dispersion. Note that blending amount is calculated based on solid portion of silicone-treated titanium dioxide. Iodine value of the oil in which the silicone-treated titanium oxide is dispersed is about 0.<br>*3: Basis LP-20H (manufactured by Nisshin OilliO Group, Ltd.) | |

## Claims

**1.** A cosmetic, comprising components below:

(a) an organic pigment,
(b) titanium dioxide particles,
(c) 4-tert-butyl-4'-methoxybenzoylmethane,
(d) a phospholipid and/or hydrogenated product thereof, and
(e) an oil,

wherein a ratio of a content of the component (d) relative to a content of the component (c) is less than 1.0.

**2.** The cosmetic according to claim 1, wherein the organic pigment comprises Red No. 104 (1).

**3.** The cosmetic according to claim 1 or 2, wherein the ratio is 0.5 or less.

**4.** The cosmetic according to claim 1 or 2, wherein a content of the 4-tert-butyl-4'-methoxybenzoylmethane is 0.1 to 10% by mass.

**5.** The cosmetic according to claim 1 or 2, wherein a content of the phospholipid and/or hydrogenated product thereof is

0.01 to 5.0% by mass.

**6.** The cosmetic according to claim 1 or 2, wherein a content of the titanium dioxide particles is 0.1 to 20% by mass.

**7.** The cosmetic according to claim 1 or 2, wherein a content of the organic pigment is 0.001 to 20% by mass.

**8.** The cosmetic according to claim 1 or 2, wherein an apparent iodine value of the oil is 6.0 to 30.0.

**9.** The cosmetic according to claim 1 or 2, which is an oily cosmetic.

**10.** The cosmetic according to claim 1 or 2, which is a lip cosmetic.

## INTERNATIONAL SEARCH REPORT

International application No.
**PCT/JP2023/021469**

**A. CLASSIFICATION OF SUBJECT MATTER**

***A61K 8/49***(2006.01)i; ***A61K 8/29***(2006.01)i; ***A61K 8/35***(2006.01)i; ***A61K 8/55***(2006.01)i; ***A61K 8/92***(2006.01)i; ***A61Q 1/04***(2006.01)i
FI: A61K8/49; A61Q1/04; A61K8/29; A61K8/35; A61K8/55; A61K8/92

According to International Patent Classification (IPC) or to both national classification and IPC

**B. FIELDS SEARCHED**

Minimum documentation searched (classification system followed by classification symbols)

A61K8/49; A61K8/29; A61K8/35; A61K8/55; A61K8/92; A61Q1/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

Mintel GNPD

**C. DOCUMENTS CONSIDERED TO BE RELEVANT**

| Category* | Citation of document, with indication, where appropriate, of the relevant passages | Relevant to claim No. |
|---|---|---|
| X<br>Y | Sun Supreme SPF 15 for Face (ID:713507). Mintel GNPD [online]. May 2007, [retrieved on 25 July 2023], Internet URL:https://www.gnpd.com<br>Ingredients, product description | 1, 3-9<br>1, 3-9 |
| X<br>Y | JP 2020-164448 A (KOSE CORP.) 08 October 2020 (2020-10-08)<br>claims, paragraph [0036], example 19 | 1-10<br>1, 3-9 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 July 2023** | **08 August 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/021469**

| Patent document cited in search report | Publication date (day/month/year) | Patent family member(s) | Publication date (day/month/year) |
|---|---|---|---|
| JP 2020-164448 A | 08 October 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2005200378 A

### Non-patent literature cited in the description

- **KODA, YOSHIO et al.** Organic Conceptual Diagrams -Fundamentals and Applications. Sankyo Publishing Co., Ltd., 1984, 11-17